# EUROPEAN PATENT APPLICATION

(11) **EP 3 828 283 A1**
(43) Date of publication of application: **02.06.2021**
(21) Application number: 19212070.7
(22) Date of filing: 28.11.2019
(51) Int. Cl.: C12Q 1/6806

(54) **AN IMPROVED SEQUENCING METHOD AND KIT**

(71) Applicant: Diagenode S.A., 4102 Seraing (BE)
(72) Inventor: BROCART, Gilles, 4130 ESNEUX (BE); SABATEL, Celine, 4550 VILLERS-LE-TEMPLE (BE); DURIEUX, Florence, 4683 VIVEGNIS (BE)
(74) Representative: Pronovem

(57) **Abstract**

The present invention is related to a Template Switch Oligo construct and its use into a ligase free diagnostic and/or sequencing method and to the kit for performing the method of the invention.

## Description

### Field of the invention

The present invention is in the field of diagnostic and/or sequencing technologies and is related to a Template Switch Oligo construct and its use into an improved ligase free diagnostic and/or sequencing method based upon Capture and Amplification by Switching technology, especially the so-called "Capture and Amplification by Tailing and Switching" (CATS) technology. The present invention is also related to a diagnostic and/or sequencing kit comprising tools for performing the method of the invention.

### Background of the invention

Sequencing methods with Capture and Amplification by Switching, especially the so called "Capture and Amplification by Tailing and Switching"(CATS) technology are ligase-free methods to produce DNA libraries for a further sequencing from low amounts (about 10 pg or more) of RNA or DNA sequences and is described in the international patent application WO2015/173402-A1.

These methods are more efficient for RNA-seq library creation than protocols using ligase by incorporating adaptors during cDNA synthesis in a single reaction tube.

In particular, these methods allows optimal sequencing of a great variety of RNA species, including sensitive, degraded, cell free RNA (cfRNAs), plasma derived RNA, non-coding RNA (ncRNA), such as miRNA or long non-coding RNA (IncRNA), exosomal RNA, rare and low input RNA sample, that are efficient markers of different diseases, such as cancers.

In addition, these rapid and easy single tube methods will minimize hands-on time, reduces possible risks of contamination and is more robust against bias factors, such as RNA modifications or secondary structure that could cause preferential enrichment, than ligation based methods.

Although the CATS library preparation technology holds significant advantages, it still presents a major limitation in its sequencing ability on ILLUMINA (ILMN) ® platforms. This limitation essentially comes from the fact that the ILLUMINA platforms read1 is starting by the template switch motif, a sequence motif of 3 to 5 positions, highly enriched in Guanosine (G).

Furthermore, the addition of at least 5 consecutive nucleotides (Poly-A tail) to the 3-terminus of the native (single stranded or double stranded) nucleic acid sequence or its fragments of the sample to be analyzed will generate technical problems, such as a difficult trimming and a loss of terminal As, during the data analysis of the reads generated.

Next Generation Sequencing (NGS) platforms like Illumina's are deciphering unknown sequence of DNA based on a Sequencing By Synthesis (SBS) principle. This principle is relying on the synthesis of a short piece of complementary DNA called a read that is sequentially detected by fluorimetry. The fluorimetric detection is possible thanks to labelled nucleotides that end up constituting the building blocks of the so called read.

In order to calibrate the different settings important for the read synthesis and the sequential detection of the nucleotides added, thus constituting the growing read, the sequencer uses the first sequencing cycles to map the location of the clusters, preferably in non-patterned flow cells at least, test image acquisition, balance the different fluorescent signals emitted by the different growing reads in the different clusters. This critical timeframe for the sequencing run is often called the template generation.

Once the template generation is completed, it is impossible for the sequencer to change its set parameters which will have a predominant impact on the rest of the sequencing run and on the quantity and quality of the data generated.

The template generation requires an even distribution of the four different bases (A, T, C and G) called at the same time across the flow cell during a sequencing cycle. Therefore, this library that is under the process of sequencing, must present a diverse distribution of the bases inside its sequence and across the different DNA fragments that will later on constitute the clusters on the flow cell. This is especially true for the first ten cycles of sequencing.

To this day, the small RNA-seq library preparation methods employing the template-switch to attach an Illumina adapter to the growing construct have been causing troubles in sequencing.

This is because the nature of the template switch construct contradicts directly the prerequisites of a library that will make a successful template generation on a (Illumina) sequencer.

Practically speaking, the 3 to 5 first sequencing cycles that are reading the template switch are highly unbalanced, and calling a high percentage (>60%) of the same base (G) for almost every clusters on the flow cell.

This severe lack of base diversity is causing important drawbacks in the sequencing output, especially numbers of reads generated in the end and the overall read quality.

### Aims of the invention

The present invention aims to provide new tools and a new ligase-free sequencing method, based upon the Capture and Amplification by Switching technology method, preferably the "Capture and Amplification by Tailing and Switching" (CATS) method above described, that do not present the drawbacks of the method and kit of the state of the art.

A first aim of the present invention is to obtain such method and tools for performing this method, that improve the nucleic acids libraries production and sequencing, especially an increase in positive reads of sensitive, degraded, chemically modified, cell free nucleic acid sequences, especially RNA sequences, possibly obtained from a single cell and therefore a higher diversity of the detected and sequenced nucleic acids, possibly involved in specific biological pathways and that could be factors of syndromes or diseases (such as inflammation or cancer) affecting bacteria, fungi, animal and plants cells, tissues, organs or species.

Another aim of the invention is to obtain such method and tools for performing this method, that are easy to use, with minimal hands-on time; that are also robust and present an improved sensitivity and excellent reproducibility.

### Summary of the invention

To overcome this major limitation of template switch-based library preparation, the inventors have designed a new library construct using CATS technology, but wherein the template switch oligo (TSO) construct used according to the invention, contains three distinct parts, each one serving a particular function.

Therefore, a first aspect of the present invention is related to a new template switch oligo (TSO) construct, also called hereafter the "improved CATS construct according to the invention" comprising from the 5' end towards the 3' end, at least :
- a primer sequence, preferably being part of the ILLUMINA ® P5 adaptor sequence, necessary for genetic material amplification, preferably for PCR amplification, flow cell clustering and sequencing primer for read one hybridization, and
- a template switch motif sequence in this TSO construct that is designed to match the overhang nucleotides added by the reverse transcriptase at the 3'end of a cDNA after first strand synthesis,
- this template switch motif sequence is linked to this primer sequence by a random linker sequence of more than 6 bases, preferably between (about) 10 bases and (about) 50 bases, that will constitute the first 10 cycles to 50 cycles of the sequencing.

Preferably, in the template switch oligo (TSO) construct according to the invention, the primer sequence is further linked by its 5' end to a label made of a chemical label group, such as the biotin.

Advantageously, the random linker sequence of the construct according to the invention comprises (about) 10, 11, 12, 13 or 14 nucleotides and is preferably composed of a more or less equal distribution of the bases A, T, C and G, i.e. from (about) 20 to (about) 30% of each base A, T, C and G, that is designed to circumvent the limitations of the template switch-based library preparation in the template generation phase.

Ultimately, since the random linker sequence present in the construct according to the invention is added to every library fragment before genetic (PCR) amplification, it can also be advantageously used as a Unique Molecular Identifier (UMI) during data analysis to eliminate PCR duplicates of a read.

Preferably, the construct according to the invention is selected from the group consisting of the sequence SEQ.ID.NO:1, the sequence SEQ.ID.NO:2 or the sequence SEQ.ID.NO:3, comprising a random linker sequence of 12 nucleotides having the sequence: NNNNNNNNNNNN,
- wherein N is any nucleotide that comprises a base selected from the group consisting of Adenosine (A), Thymidine (T), Guanine (G) or Cytosine (C),
- wherein the random linker sequence comprises more or less about 25% of each base A,T,C and G, and
- wherein the sequence rNrGrGrG is the consensus template switch sequence, rN is a nucleoside or any ribonucleotide of a RNA sequence and rG is a specific nucleoside : a ribonucleotide of the base guanine (G).

Advantageously, the sequence SEQ.ID.NO:1 comprises or preferably consist of the following sequence: 5' (biotin)-GTTCAGAGTTCTACAGTCCGACGATCNNNNNNNNNNNNrNrGrGrG 3' and SEQ.ID.N0:2 comprises or preferably consist of the following sequence: 5' (biotin)-TTCCCTACACGACGCTCTTCCGATCTNNNNNNNNNNNNrNrGrGrG 3' correspond respectively to the construct according to the invention, wherein the primer sequence is present in the known illumina ® adaptor sequence with dual indexing (CATS-ILMN (Truseq HT) and without dual indexing (CATS-IMN) (truSeqsmRNA)) and the sequence SEQ.ID.NO:3 comprises or preferably consist of the following sequence: 5' (biotin)-GAACGACATGGCTACGATCCGATCTTNNNNNNNNNNNNrNrGrGrG 3' is the construct according to the invention, wherein the primer sequence is present in the known MGI ® adaptor sequence (CATS-MGI).

Preferably, one or more of the sequences SEQ.ID.NO:1, SEQ.ID.NO:2 and/or SEQ.ID.NO:3 is (are) also linked by the 5' end of the primer sequence to a chemical label group, such as biotin.

The unique design of this template switch oligo, like it has been proven in sequencing, allows removing the barriers caused by the direct sequencing of the template switch motif in small RNA-seq applications using a template switch-based library preparation. Using this unique design of the new construct according to the invention, one might expect to have a sequencing run falling completely into ILLUMINA ® specifications without using more than 1% phiX spiked-in which hasn't been possible by the past in the previously stated conditions (direct sequencing of the template switch motif).

The present invention is also related to a diagnostic and/or sequencing method, preferably high throughput diagnostic and/or sequencing method and possibly to a computer-implemented method performed under the control of processor executing instructions, which is a diagnostic and/or sequencing method of a nucleic acid strand sequence as well as tools, preferably included into a kit, for performing this method, this sequencing method comprising at least (or is consisting of) the steps of, preferably the following consecutive steps of:
- providing a sample, especially liquid or solid biopsies, such as a blood sample, preferably the serum or plasma, a tissue sample, a fossil, a single cell sample or even targeted compartments of cells, such as the nucleus, the endoplasmic reticulum, ...this sample comprising a native single stranded nucleic acid sequence or native double stranded nucleic acid sequence,
- possibly fragmenting the native single stranded nucleic acid sequence or native double stranded nucleic acid sequence, into smaller nucleic acid sequence fragments,
- possibly denaturing the native double strand nucleic acid sequence(s),
- possibly end-repairing the native nucleic acid sequences,
- preferably adding at least 5, preferably at least 10, at least 15, at least 20, at least 25, at least 30, at least 35, but preferably less than 100, less than 75, less than 50 consecutive nucleotides to the 3' terminus of the native single stranded or native double stranded nucleic acid sequence or their fragments, preferably a poly(A) sequence.
- hybridizing a priming oligonucleotide sequence complementary to the added nucleotide sequence and synthesizing a cDNA sequence with a template dependent DNA polymerase to obtain a double stranded nucleic acid sequence,
- hybridizing to the generated double stranded nucleic acid sequence, a template switching motif sequence of a Template Switching Oligonucleotide (TSO), this template switching motif sequence being further linked in 3' to at least 6 base random sequence, preferably a random sequence comprised between (about) 10 bases and (about) 50 bases, preferably the Template Switching Oligonucleotide sequence of the invention,
- extending the 3' end of the cDNA strand to synthesize a double stranded nucleic acid sequence, wherein one strand of the nucleic acid sequence comprises the priming oligonucleotide and a cDNA sequence that is complementary to the single stranded nucleic acid sequence and to the Template Switching Oligonucleotide sequence. When the reverse transcriptase reaches the 5' end of the nucleic acid sequence, it switches the template and continues DNA synthesis over the Template Switching Oligonucleotide (TSO); the Template Switching Oligonucleotide containing at least three, preferably four 3' terminal ribonucleotides x (rX) facilitates the template switching and carries the adaptor sequence,
- performing genetic amplification steps, preferably PCR amplification steps,
- possibly adding a splint oligo sequence that hybridizes to adapter DNA sequences which are ligated to form a circle and adding an exonuclease to remove all remaining single stranded and double stranded DNA products to collect only circular DNA template,
- performing a base sequencing, and
- obtaining by nanopore sequencing or imaging an identification of each nucleotide of the native nucleic acid sequence, preferably upon a high resolution CCD camera.

In the method of the invention, the synthesized double stranded nucleic acid sequences present a length preferably comprised between (about) 150 nucleotides and (about) 600 nucleotides, more preferably between (about) 200 nucleotides and (about) 500 nucleotides.

According to the invention, the native single stranded nucleic acid sequence or native double stranded nucleic acid sequence is selected from the group consisting of normal DNA or RNA sequences, fragmented and/or bisulfite-converted DNA sequence, mRNA sequence, miRNA sequence, small RNA sequence, piRNA sequence, bisulfite-converted RNA or a mixture thereof.

In the method according to the invention, the at least 5, 10, 15, 20, 25, 30, 35 consecutive identical nucleotides are preferably selected from the group consisting of ribonucleotides, desoxy-ribonucleotides or didesoxy-ribonucleotides of A, T, C, G or U, that are preferably added by an enzyme selected from the group consisting of a poly(A)-polymerase, poly(U)-polymerase, poly(G)-polymerase, terminal transferase, DNA ligase, RNA ligase and the dinucleotides and the trinucleotides RNA ligases.

Preferably, the method of the invention is also advantageously improved prior to base sequencing step, by the addition of the step of :
- obtaining DNA clusters or nanoballs (DNBs) preferably by performing a rolling circle replication of the synthesized stranded nucleic acid sequence and fixing the DNA clusters or DNA nanoballs (DNBs) on a patterned array flow cell.

Another aspect of the invention concerns an apparatus or a sequencing kit for performing the method of the invention, this apparatus or kit comprising (or is consisting of) the following reagents present in suitable vials:
- a reagent capable of adding nucleotides to the 3-terminus of a single stranded nucleic acid,
- reagents for a genetic material amplification, preferably reagents for performing a PCR amplification, a reverse transcriptase enzyme,
- a priming oligonucleotide,
- possibly a fluorophore and
- a template switching motif sequence which is linked to an at least 6 bases random sequence, preferably between (about) 10 bases and (about) 50 bases, more preferably the Template Switching Oligonucleotide (TSO) according to the invention,

Advantageously, the apparatus or kit according to the invention may further comprise:
- possibly a rolling circle replication enzyme, preferably the Phi 29 DNA polymerase and one or more of the following elements:
- reagents for cyclization,
- a patterned flow cell,
- a template independent DNA or RNA polymerase and a blocking nucleotide, such as 3d-NTP, 3-Me-NTP and ddNTP,
- a system allowing for dual indexing, and
- possibly (written) instructions for performing the method steps of the invention.

The apparatus according to the invention can be a sequencing device configured to perform a computer-implemented sequencing method of a biological sample under the control of a processor executing instructions, this device comprising a memory device comprising executable application instructions stored therein and a processor configured to execute applications instructions stored in the memory device, these application instructions being able to perform or to control one or more steps of the claimed method of the invention.

In the method, apparatus and kit according to the invention, the priming oligonucleotide preferably comprises the nucleotide sequence disclosed in the claims 9 to 12 and in the claims 19 and 20 of international patent application WO2015/173402, incorporated herein by reference.

Advantageously, in the method, apparatus and kit according to the invention, the rolling cycle amplification is obtained by addition of a sufficient amount of the Phi 29 DNA polymerase, this enzyme allows the production of clusters, concatemers or DNA nanoballs (DNBs) into a long single stranded DNA sequence comprising several head-to-tail copies of the circular template, wherein the resulting nanoparticle self assembles into a tight ball of DNA. This polymerase replicates the looped DNA and when it finishes one circle, it does not stop-it, but it continues the replication by peeling off its -previously copied DNA. This copying process continues over and over, forming the DNA cluster or DNA nanoball, as a large mass of repeating DNA to be sequenced all connected together.

Preferably, in the method, apparatus and kit according to the invention the patterned array flow cell is a silicon wafer coated with silicon dioxide, titanium, hexamethyldisilazane (HDMS) and a photoresist material and each DNA nanoball selectively binds to the positively-charged aminosilane according to the pattern.

Advantageously, in the method of the invention, the sequencing is obtained by adding dNTP incorporated by polymerase, each dNTP being conjugated to a particular label, preferably a label being a fluorophore or dye and containing a termination blocking addition extension, wherein unincorporated dNTPs are washed, wherein image is captured, wherein dye and terminator are cleaved and wherein these steps are repeated until sequencing is complete. In addition, in the method of the invention, the added fluorophore is excited with a laser that sends light of a specific wavelength, fluorescence emission from each DNA cluster or DNA nanoball is captured on high resolution CCD camera and wherein color of each DNA cluster or each DNA nanoball corresponds to a base to the interrogative position, so that a computer can record a base position information.

A last aspect of the invention concerns the use of the apparatus, the kit or the method according to anyone of the preceding claims, for sequencing or expression analysis, for cloning labelling, for the identification of genes or mutation(s), in personalized medicine, therapy monitoring, prediction, prognosis, early detection of human or animal disease or forensic science, in the analysis of infectious diseases and genomes of viruses, bacteria, fungi, animals or plant, including their derived cells, in the characterization of plants, fruits, breeding checks detection of plants or fruits diseases.

### Definitions

All literature and similar material cited in this application, including, but not limited to, patents, patent applications, scientific articles, books and web pages are expressly incorporated by reference in their entirety to the description of the present invention.

Unless otherwise defined, all terms used in disclosing the invention, including technical and scientific terms, have the meanings as commonly understood by one of ordinary skilled in the art in the invention field.

.As used in this specification and claims, the singular forms "a", "an" and "the" include singular or plural referents, unless the content clearly dictates otherwise.

The terms "comprising", "comprises" and "comprised of" are synonymous with "including", "includes" or "containing" and ," contains" are either inclusive or open ended and do not exclude additional, non-recited members, elements or method steps.

The terms "one or more" or "at least one", is clear per se and encompass a reference to any of these members, which means any two or more of the members and up to all members.

The term "about" as used herein, when referring to a measurable value such as an amount of a compound, dose, time and the like is meant to encompass 10%, 9%, 8%, 7%, 6%, 5%, 4%, 3%, 2%, 1%, 0.5% or even 0.1% of the specified amount or value.

As used in the specification and claims, the terms "nucleic acid(s)" comprises polymeric or oligomeric macromolecules, including DNA ((deoxyribonucleic acid) and RNA (ribonucleic acid) known as nucleotides. Comprising bases selected from the group consisting of Adenine (A), Thymine (T), Cytosine (C), Guanine (G) and Uracil (U).

The term "nucleoside" refers to a base linked to ribose cycle by a beta-N9 glycosidic link.

The terms "single stranded nucleic acid" (ss nucleic acid) refer to a nucleic acid which consist of only one polynucleotide or oligonucleotide strand. In contrast, a "double stranded nucleic acid" (ds nucleic acid) consist of two polynucleotide or oligonucleotide strands wherein the majority of the nucleotides are paired according to known pairing rules.

The term "sample" refers to a part or piece of a tissue, organ or individual, typically being smaller than such tissue, organ or individual, intended to represent the whole of the tissue, organ or individual.

The terms "genetic amplification" is a biochemical technology used in molecular biology for many years to amplify by primers sequences a single or few copies of a piece or portion of DNA by replication and copy across several orders of magnitude, generating thousands to millions of copies of a particular DNA Sequence. The most known genetic amplification technology is the so called "Polymerase Chain Reaction" or PCR, as described in US patent 4, 683, 195-B2 and US 4, 683, 202-B2, using two primers sequences and the heat stable DNA polymerase, such as the Taq polymerase obtained from bacterium *Thermus aquatica* allowing thermal cycling.

The term "primer" refers to a oligonucleotide sequence, usually comprising between about 12 nucleotides and about 25 nucleotides, hybridizing specifically to a target sequence of interest and which functions as a substrate onto which nucleotides can be polymerized by a polymerase.

The terms "Template Swich Oligo "refer to an oligo that hybridizes to untemplated C nucleotides added by a reverse transcriptase during reverse transcription.

The terms "the primer sequence of the Illumina P5 (and P7) adaptor sequence" are known and correspond respectively for single indexing to:
- P5: 5' AAT GAT ACG GCG ACC ACC GA 3' and
- P7: 5' CAA GCA GAA GAC GGC ATA CGA GAT 3'
- 
-                                        P5:                                        5’
AATGATACGGCGACCACCGAGATCTACACGTTCAGAGTTCTACAGT
CCGACGATC 3’ and
- 
- P7: 5'
GATCGGAAGAGCACACGTCTGAACTCCAGTCAC[i]ATCTCGTATGCCGTCTTC
TGCTTG 3' and
for dual indexing to :
- 
-                                        P5:                                        5'
AATGATACGGCGACCACCGAGATCTACAC[i5]ACACTCTTTCCCTACACGACG
CTCTTCCGATCT 3' and
- P7: 5' GATCGGAAGAGCACACGTCTGAACTCCAGTCAC[i7]ATCTCGTATGCCGTCTT CTGCTTG 3' refer to amplification primers pairs being a universal primer extension primers pairs as described in the international patent applications WO2007/010251, WO2006/064199, WO2005/065814, WO2015/106941 and WO200/18957, all incorporated herein by reference.

The present invention will be described hereafter in the following examples in reference to the enclosed drawings and presented as non-limiting illustrations of the present invention.

### Short description of the drawings

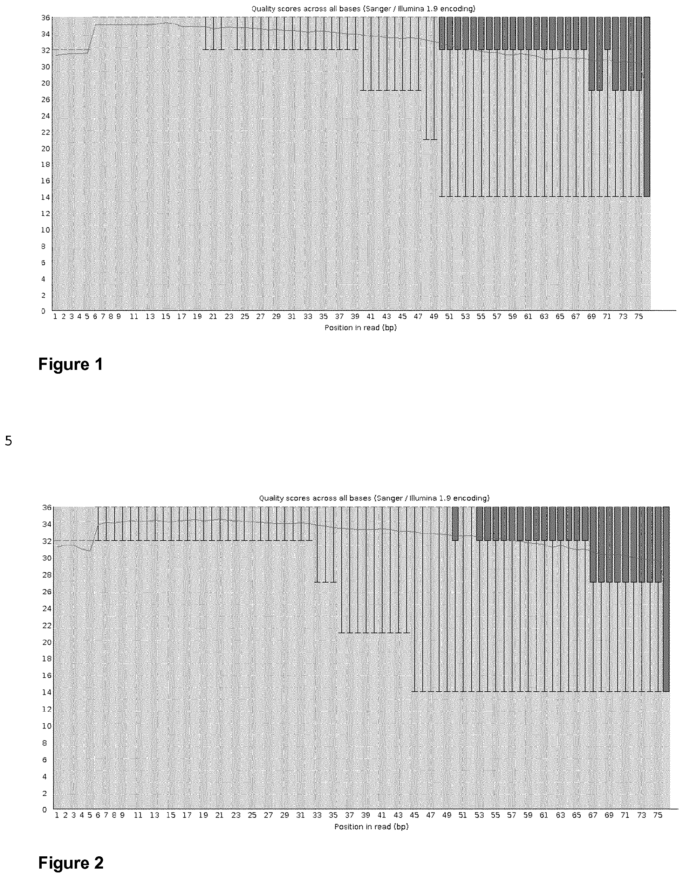

The enclosed figures 1 to 4 present results of comparative analysis of the quality and quantity of data generated with applied with CATS method of the invention (when the improved CATS construct includes a random sequence) and with CATS method of the state of the art (when the standard CATS construct does not include the random sequence).
Figures 1 and 2 represent per base sequence quality results in a NextSeq 550 run obtained respectively with the improved CATS construct according to the invention and with the standard CATS construct of the state of the art.
Figures 3 and 4 represent per base sequence quality results in a HiSeq 4000 lane (5% phiX) obtained respectively with the standard CATS construct of the state of the art and with the improved CATS construct according to the invention.

### Examples

### Comparative analysis of the quality and quantity of data generated from a sequencing run between the CATS-UMI construct and the standard CATS construct.

### 1. Output of the sequencing run

The two CATS constructs have been sequenced independently on two NextSeq 550 runs. The improved CATS construct, CATS-UMI, has been sequenced in a NextSeq 550 HO flow cell in SE50 mode with 3% phiX and following Illumina's recommendations in terms of clusters density. The output of this run is exactly 407 million reads. This is above the maximum (400 million) output described by Illumina in its documentation.
Percentage-wise, the output of the run is 101, 75%.

On the other hand, the standard CATS construct has been sequenced in a NextSeq 550 MO flow cell in SE50 mode with 20% phiX to prevent any run failure due to the incompatibility of the template switch being read in the first sequencing cycles.

Therefore, it is already logical that the maximum output of the run will never be reached as the library pool has been spiked in to 20% with an external phiX library to overcome the low complexity start in the sequencing. Even though, the maximum for this run could only be 80% of the true maximum (taking into account 20% phiX), the run didn't even reach that 80% bar.

According to Illumina protocol, a NextSeq 550 MO (mid-output) run is able to yield maximum 130 million reads. In this situation, this maximum would have to be decreased down to its 80% if we consider that the clustering of the CATS libraries is going perfectly fine. Thus, it could have been expected a maximum output for the pool of CATS libraries to be 104 million of reads. The results of this run was only 94, 2 million of reads, meaning 90, 58% of what could be have expected at most.

Therefore, sequencing using the standard CATS construct as opposed to the improved CATS-UMI represents two flaws in terms of sequencing output:
a. Using an important phiX spike-in in the library pool thus already decreasing the number of reads a flow cell will generate for CATS libraries;
b. And even though the sequencing run accounts for a significant part of phiX, the space left in output for CATS libraries is not completely filled. This might be also a negative consequence of the CATS clusters beginning with the template switch motif, thus likely hindering the template generation phase of the sequencing.

A confirmation of the better capability of the improved CATS construct (CATS-UMI) according to the invention to generate a higher output has been obtained while comparing the two constructs in a HiSeq 4000 SE50 sequencing, following Illumina's recommendations. The standard CATS construct of the state of the art has been sequenced with 5% phiX while the improved construct was sequenced only with 0, 5% phiX for run monitoring purpose.

The difference in output is rather surprising:
- Sequencing lane with the standard CATS construct yielded 144 million of reads passing filter out of -312 million reads (maximum described in Illumina's documentation);
- Sequencing lane with the improved construct yielded 314, 6 million of reads passing filter out of -312 million reads (maximum described in Illumina's documentation).

The improved construct is thus able to reach the maximum output and even surpassing it a little bit as in NextSeq sequencing, -100%, whereas the standard construct is rather working poorly and giving out an output of only 46% of the maximum.

### 2. Quality of the sequencing run

Comparing the 'per base quality' of the two CATS construct depicted in section 1. In a NextSeq run is somehow biased because the standard CATS construct is rescued by a significant spike in of phiX of 20%. Therefore, the 'per base quality' of a CATS library is better thanks to a balanced spike-in such as phiX that is positively impacting the template generation phase among other parameters.

As figures 1 and 2 are showing, the 'per base sequence quality' between the two construct is not obvious. In order to have a better comparison where the phiX spike-in isn't playing such a big role, the two constructs have also been sequenced on another Illumina sequencer that is less sensitive to low complexity pattern in the beginning of the read.

The standard CATS construct of the state of the art has been sequenced in a HiSeq 4000 lane with 5% phiX (only) following Illumina's specifications whereas the improved CATS-UMI construct according to the invention has also been sequenced in a HiSeq 4000 lane but with only 0,5% phiX spike-in (for run monitoring purpose).

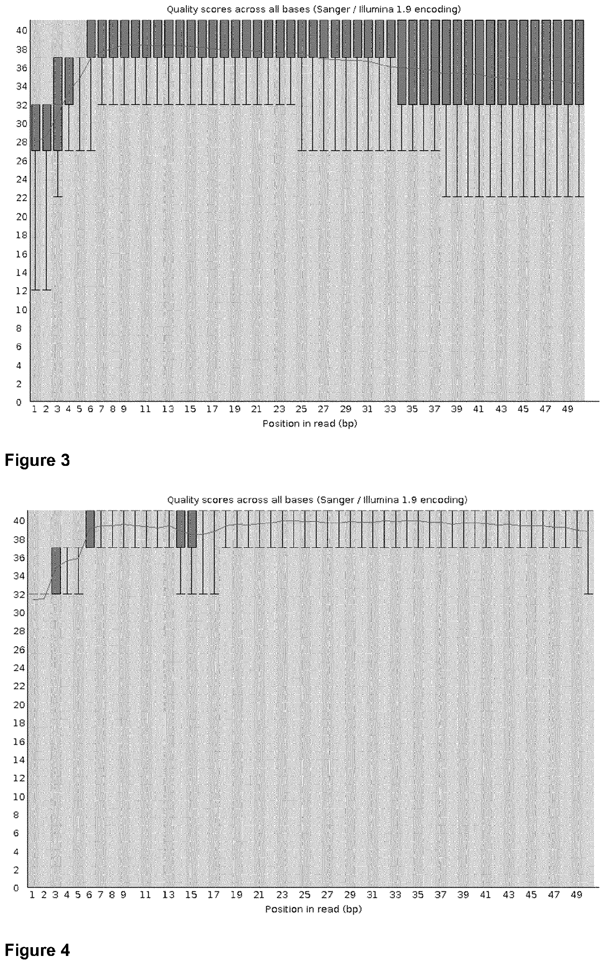

As figures 3 and 4 are showing, the difference in 'per base sequence quality' becomes quite clear.

Once the support of the phiX is retrieved from the run, the somehow real 'per base sequence quality' can be observed. When comparing both constructs in figures 3 and 4, it can easily be observed that adding a random sequence before the template switch in the improved CATS construct according to the invention is highly beneficial in terms of data quality.

### 3. Conclusion

Based on the results presented herein, the skilled person of the art can observe that the improved CATS construct according to the invention containing a random sequence in front of the template switch motif during read1 is prone to generate more data output from a sequencing run as well as overall better data quality.

Application Project
-------------------
<120> Title : An improved  sequencing method and kit
<130> AppFileReference : BPDIAG0008EP01
<140> CurrentAppNumber :
<141> CurrentFilingDate : ____-__-__

Sequence
--------
<213> OrganismName : Artificial sequence
<400> PreSequenceString :
gttcagagtt ctacagtccg acgatcnnnn nnnnnnnnng gg                    42
<212> Type : DNA/RNA
<211> Length : 42
      SequenceName : SEQ.ID.NO:1
      SequenceDescription :

Feature
-------
Sequence: SEQ.ID.NO:1:
<221> FeatureKey : N_region
<222> LocationFrom : 27
<222> LocationTo : 39
      Other Information :
      CDSJoin : No

Sequence
--------
<213> OrganismName : Artificial sequence
<400> PreSequenceString :
ttccctacac gacgctcttc cgatctnnnn nnnnnnnnng gg                    42
<212> Type : DNA/RNA
<211> Length : 42
      SequenceName : SEQ.ID.NO:2
      SequenceDescription :

Sequence
--------
<213> OrganismName : Artificial sequence
<400> PreSequenceString :
gaacgacatg gctacgatcc gatcttnnnn nnnnnnnnng gg                    42
<212> Type : DNA/RNA
<211> Length : 42
      SequenceName : SEQ.ID.NO:3
      SequenceDescription :

Sequence
--------
<213> OrganismName : Artificial sequence
<400> PreSequenceString :
aatgatacgg cgaccaccga                                             20
<212> Type : DNA
<211> Length : 20
      SequenceName : SEQ.ID.NO:4
      SequenceDescription :

Sequence
--------
<213> OrganismName : Artificial sequence
<400> PreSequenceString :
caagcagaag acggcatacg agat                                        24
<212> Type : DNA
<211> Length : 24

SequenceName : SEQ.ID.NO:5
SequenceDescription :

Sequence
--------
<213> OrganismName : Artificial sequence
<400> PreSequenceString :
aatgatacgg cgaccaccga gatctacacg ttcagagttc tacagtccga cgatc 55

<212> Type : DNA
<211> Length : 55
SequenceName : SEQ.ID.NO:6
SequenceDescription :

Sequence
--------
<213> OrganismName : Artificial sequence
<400> PreSequenceString :
gatcggaaga gcacacgtct gaactccagt cacatctcgt atgccgtctt ctgcttg 57

<212> Type : DNA
<211> Length : 57
SequenceName : SEQ.ID.NO:7
SequenceDescription :

Sequence
--------
<213> OrganismName : Artificial sequence
<400> PreSequenceString :
aatgatacgg cgaccaccga gatctacaca cactctttcc ctacacgacg ctcttccgat 60
ct 62
<212> Type : DNA
<211> Length : 62
SequenceName : SEQ.ID.NO:8
SequenceDescription :

Sequence
--------
<213> OrganismName : Artificial sequence
<400> PreSequenceString :
gatcggaaga gcacacgtct gaactccagt cacatctcgt atgccgtctt ctgcttg 57

<212> Type : DNA
<211> Length : 57
SequenceName : SEQ.ID.NO:9
SequenceDescription :

## Claims

1. A Template Switching Oligonucleotide construct for sequencing and comprising from a 5' end towards a 3' oriented end:
- a primer sequence
- a template switching motif sequence which is linked to the said primer sequence by a random linker sequence of more than 6 bases.

2. The construct of claim 1, wherein the random linker sequence comprises more than 10 bases, preferably between 10 bases and 50 bases.

3. The construct according to the claim 1 or to the claim 2, wherein the random linker sequence is composed of more or less equal distribution of bases.

4. The construct according to any one of the preceding claims 1 to 4, wherein the primer sequence is part of the ILLUMINA P5 adaptor sequence.

5. The construct according to the claim 5, which consists or comprises the sequence SEQ.ID.NO:1 or the sequence SEQ.ID NO: 2.

6. The construct according to any one of the preceding claims 1 to 4, which consists of or comprises the sequence SEQ.ID.NO:3

7. A sequencing method, preferably a high throughput sequencing method of a nucleic acid sequence comprising the steps of:
- providing a sample, single cell sample, comprising a native single stranded nucleic acid sequence or native double stranded nucleic acid sequence,
- possibly fragmenting the native single stranded nucleic acid sequence or native double stranded nucleic acid sequence into smaller nucleic acid sequence fragments,
- possibly denaturing the native double strand nucleic acid sequence(s)
- possibly end-repairing the native nucleic acid sequences,
- hybridizing a priming oligonucleotide sequence complementary to the added nucleotide sequence and synthesizing a cDNA sequence with a template dependent DNA polymerase to obtain a double stranded nucleic acid sequence,
- hybridizing to the said generated double stranded nucleic acid sequence, a template switching motif sequence, wherein the template switching motif sequence is linked to a random sequence made of at least 6 bases, preferably of between 10 bases and 50 bases,
- extending the 3' end of the cDNA strand to synthesize a double stranded nucleic acid sequence, wherein one strand of the nucleic acid sequence comprises the priming oligonucleotide and a cDNA sequence that is complementary to the single stranded nucleic acid sequence and to the template switching oligonucleotide sequence,
- performing a genetic amplification, preferably a PCR genetic amplification,
- possibly adding a splint oligo sequence that hybridize to adapter DNA sequences which are ligated to form a circle and adding an exonuclease to remove all remaining single stranded and double stranded DNA products to collect only circular DNA template, and
- performing a base sequencing.

8. The method according to the claim 8, which further comprises the step of adding at least 5 consecutive nucleotides to the 3-terminus of the native single stranded or native double stranded nucleic acid sequence or their fragments before the hybridizing step, preferably the at least 5 consecutive identical nucleotides are selected from the group consisting of ribonucleotides, desoxy-ribonucleotides or didesoxy-ribonucleotides of A, T, C, G or U, preferably a poly-A sequence of at least 5 nucleotides, preferably of at least 10 nucleotides and wherein the at least 5 consecutive nucleotides are preferably added by an enzyme selected from the group consisting of a poly(A)-polymerase, poly(U)-polymerase, poly(G)-polymerase, terminal transferase, DNA ligase, RNA ligase and the dinucleotides and the trinucleotides RNA ligases.

9. The method according to the claim 7 or to the claim 8, wherein the template switching motif sequence linked to the random sequence, comprises or consist of the Template Switching Oligonucleotide construct according to any one of the preceding claims 1 to 6.

10. The method according to any one of the preceding claims 7 to 9, wherein the sequencing is obtained by adding dNTP incorporated by polymerase, each dNTP being conjugated to a label, preferably the label being a fluorophore and containing a termination blocking addition extension, wherein unincorporated dNTPs are washed, wherein image is captured, wherein dye and terminator are cleaved and wherein these steps are repeated until sequencing is complete and, wherein the fluorophore is preferably excited with a laser that emits light of a specific wavelength, wherein fluorescence emission from each DNA cluster is preferably captured on high resolution CCD camera, wherein a color of each detected DNA cluster corresponds preferably to a base to the interrogative position and wherein a computer records preferably base position information.

11. The method according to any one of the preceding claims 7 to 10, wherein the synthesized double stranded nucleic acid sequences present a length comprised between 150 nucleotides and 500 nucleotides.

12. The method according to any one of the preceding claims 7 to 11, wherein the native single stranded nucleic acid sequence or native double stranded nucleic acid sequence is selected from the group consisting of fragmented and/or bisulfite-converted DNA sequence, mRNA sequence, miRNA sequence, small RNA sequence, piRNA sequence, bisulfite-converted RNA or a mixture thereof.

13. A sequencing kit comprising:
- a reagent capable of adding nucleotides to the 3-terminus of a single stranded nucleic acid,
- an end-repair enzyme,
- reagents for a genetic material amplification, preferably a PCR amplification,
- a reverse transcriptase enzyme,
- a priming oligonucleotide,
- possibly a fluorophore and
- a template switching oligonucleotide, wherein the said template switching motif sequence is linked to an at least 6 bases or 10 bases random sequence, preferably a random sequence comprising between 10 bases and 50 bases, or the template Switching Oligo construct according to any one of the preceding claims 1 to 6.

14. The kit according to the claims 13, wherein the reagent is a template independent DNA or RNA polymerase and a blocking nucleotide, such as 3d-NTP, 3-Me-NTP and ddNTP.

15. Use of the construct, the method or the kit according to anyone of the preceding claims, for sequencing or expression analysis, for cloning labelling, for an identification of genes or mutation(s), in personalized medicine, therapy monitoring, prediction, prognosis, early detection of human or animal disease or forensic science, in the analysis of infectious diseases and genomes of viruses, bacteria, fungi, animals or plant, including their derived cells and in the characterization of plants, fruits, breeding checks detection of plants or fruits diseases.
